# EUROPEAN PATENT SPECIFICATION

(11) **EP 2 661 288 B1**
(45) Date of publication and mention of the grant of the patent: **01.04.2020**
(21) Application number: 12732457.2
(22) Date of filing: 04.01.2012
(51) Int. Cl.: A61M 1/00, A61B 17/221, A61B 17/50, A61B 17/3205

(54) **VARIABLE LOOP SNARE**
SCHLINGE MIT VARIABLER SCHLEIFE
ANSE À BOUCLE VARIABLE

(30) Priority: 04.01.2011 US 201161429691 P
(43) Date of publication of application: 13.11.2013
(73) Proprietor: Merit Medical Systems, Inc., South Jordan, UT 84095 (US)
(72) Inventor: LAMPROPOULOS, Fred, Salt Lake City, Utah 84117 (US); MOTTOLA, Jim, Salt Lake City, Utah 84195 (US); FLYGARE, Mark, Media, PA 19063 (US); BYRNE, Paul, Tuam (IE)
(74) Representative: Ricker, Mathias
(86) International application number: PCT/US2012/020208
(87) International publication number: WO 2012/094408

(56) References cited:
- JP-A- 2005 270 464
- US-A- 5 171 233
- US-A- 5 562 678
- US-A- 5 860 987
- US-A1- 2009 118 760
- US-B1- 6 193 672

## Description

### TECHNICAL FIELD

The present disclosure relates generally to medical devices configured for use within the human body. Specifically, the present disclosure relates to a snare device comprising a loop configured to adjust to one or more sizes.

### BRIEF DESCRIPTION OF THE DRAWINGS

The embodiments disclosed herein will become more fully apparent from the following description and appended claims, taken in conjunction with the accompanying drawings. These drawings depict only typical embodiments, which will be described with additional specificity and detail through use of the accompanying drawings in which:
Figure 1A is a perspective view of a variable loop snare device.
Figure 1B is a perspective view of the snare device of Figure 1A, oriented to show the position of the snare loop relative to the elongate shaft.
Figure 2A is a partial cut-away view of another embodiment of a variable loop snare device in a first configuration.
Figure 2B is a partial cut-away view of the snare device of Figure 2A in a second configuration.
Figure 3A is a side view of another embodiment of a snare device, in a first configuration, disposed within a cross-sectional view of a body lumen.
Figure 3B is a side view of the snare device of Figure 3A, in a second configuration, disposed within a cross-sectional view of the body lumen of Figure 3A.
Figure 3C is a side view of the snare device of Figures 3A and 3B, in a third configuration, disposed within a cross-sectional view of the body lumen of Figures 3A and 3B.

### BACKGROUND

Snares may be utilized as part of certain therapies, including minimally invasive therapies. For instance, a snare may be used to capture a foreign object located within a body lumen, such as within the vasculature. Snares of various sizes may be utilized depending on, for example, the type or nature of the therapy, the location of the therapy within the body, and so on.

JP 2005 270464 A discloses a catheter for removing and sucking foreign matters inside a blood vessel, wherein the catheter is configured to strip an embolic material from the interior of a blood vessel without damaging the blood vessel. For this purpose, the catheter comprises a wire-like member provided with a loop part, wherein the surface of the loop part is to be provided with lubricity when wetted and wherein said loop part is configured to be movable.

US 2009/0118760 A1 discloses a vascular filter retrieval device, which comprises a catheter and a loop for engaging a vascular filter. The loop is extendable from the proximal end of the catheter and comprises a resilient wire. The catheter is configured to be introduced into the vena cava of a patient and is further arranged for surrounding said vascular filter.

US 6,193,672 B1 discloses a lavage for suction and removal of body tissues in a patient. The lavage comprises an outer cannula and an inner cannula, to which a tip instrument can be attached, wherein the tip instrument includes a loop portion and a pair of legs. The loop portion expands radially outward when the outer cannula is in a retracted position.

### SUMMARY

As further described below, the present disclosure relates to snare loops which may be configured to be disposed such that a single loop is capable of assuming a variety of sizes. The invention is defined by claim 1. Preferred embodiments of the invention are defined by the dependent claims.

### DETAILED DESCRIPTION

In some instances a variable size snare loop may simply certain medical procedures or therapies by allowing a practitioner to resize the snare loop without completely removing the snare device and inserting an alternate snare device. For example, when utilizing a variable loop snare a practitioner may simply resize a variable snare loop - larger or smaller - during the course of the procedure; obviating the need to withdraw the snare loop and associated shaft and replace it with a snare loop of a different size.

It will be readily understood with the aid of the present disclosure that the components of the embodiments, as generally described and illustrated in the figures herein, could be arranged and designed in a variety of configurations. Thus, the following more detailed description of various embodiments, as represented in the figures, is not intended to limit the scope of the disclosure, but is merely representative of various embodiments. While the various aspects of the embodiments are presented in drawings, the drawings are not necessarily drawn to scale unless specifically indicated.

As used herein a "snare device" generally refers to an elongate instrument comprising a snare loop. Snare devices, as described herein, may further include additional components, such as an outer sheath, for example, though these components are not required. Additionally, a "snare loop" need not be arcuate or circular in shape, but may be any shape enclosed by a loop a material, for example by a wire.

Furthermore, snare loops described as having the same "general shape," refer to loops which enclose similar, though not necessarily identical, shapes regardless of the size of the shape. Thus, a snare loop of any shape may be scaled up or down and still be described as having the same "general shape" as the original snare loop. Moreover, two loops of different sizes may have the same "general shape" notwithstanding minor changes or variations in the shape, disposition, orientation, or proportion of the snare loops as they are scaled up or down. For example, a loop comprising two substantially straight portions joined by a substantially arcuate portion may be described as having the same general shape as a larger or smaller version of the loop in which the straight portions comprise a greater or lesser proportion of the entire loop, as compared to the proportions of the original loop. Additionally, in some instances, "scaling up" or "scaling down" the loop size may result in loops which enclose relatively larger or smaller areas, respectively.

The phrases "connected to," "coupled to," and "in communication with" refer to any form of interaction between two or more entities, including mechanical, electrical, magnetic, electromagnetic, fluid, and thermal interaction. Two components may be coupled to each other even though they are not in direct contact with each other. For example, two components may be coupled to each other through an intermediate component.

The terms "proximal" and "distal" refer to opposite ends of a medical device. As used herein, the proximal end of a medical device is the end nearest a practitioner during use, while the distal end is the opposite end. For example, the proximal end of a snare device refers to the end nearest the practitioner when the snare device is in use.

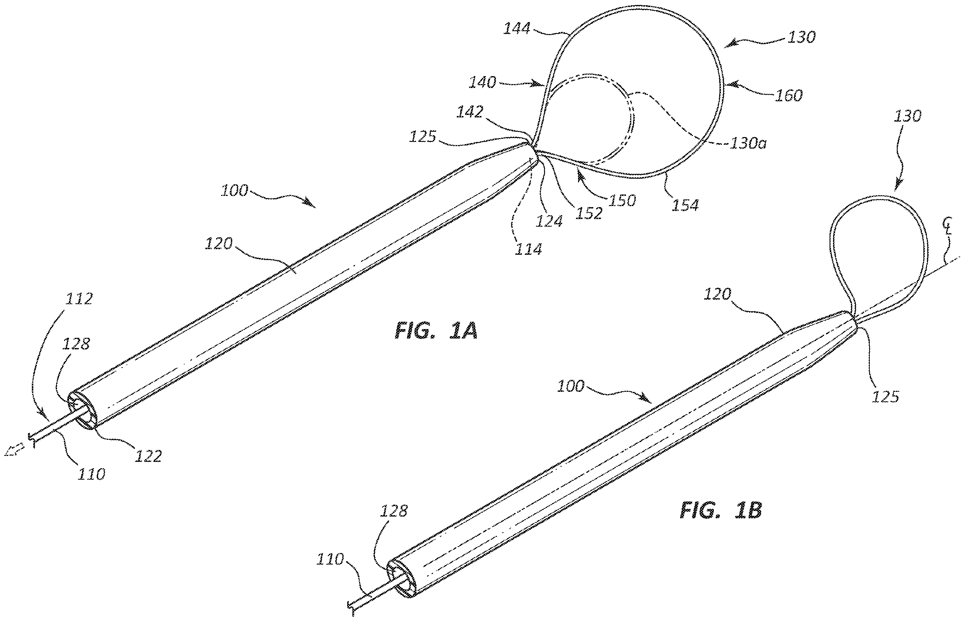

Figure 1A is a perspective view of a variable loop snare device 100. In this exemplary embodiment, the snare device 100 comprises an elongate shaft 110 disposed within an outer sheath 120. The elongate shaft 110 has a distal end 114 and a proximal end 112, one or both of which may extend from the outer sheath 120. As shown in the illustrated embodiment, the outer sheath 120 may comprise a lumen 128, and the elongate shaft 1 10 may be disposed with the lumen 128. In the illustrated embodiment, the proximal end 112 of the elongate shaft 110 extends from the proximal end 122 of the outer sheath 120, while the distal end 114 of the elongate shaft 110 is disposed within the lumen 128 of the outer sheath 120. The elongate shaft 110 may be longitudinally displaceable within the lumen 128 of the outer sheath 120, allowing, for example, the distal end 114 of the elongate shaft 110 to be displaced such that it extends from the distal end 124 of the outer sheath 120. The outer sheath 120 may further include a distal opening 125 in communication with the lumen 128 of the outer sheath 120. In some embodiments the distal opening 125 may be disposed at the distal end 124 of the outer sheath 120, while in other embodiments it may be disposed through a sidewall of the outer sheath 120 at a location adjacent the distal end 124. The elongate shaft 110 may be configured to extend from the distal opening 125 in some configurations.

A snare loop 130 may be coupled to the distal end 114 of the elongate shaft 110. In the illustrated embodiment, the snare loop 130 is deployed from - meaning it is positioned such that it extends from - the distal opening 125 of the outer sheath 120. The snare loop 130 shown in the illustrated embodiment may be subdivided into a first leg 140, a second leg 150, and a bridge member 160. These component subdivisions may refer to different portions of a integrally formed loop, or may be separate elements coupled together to form the snare loop 130.

The legs 140, 150 may define proximal 142, 152 and distal 144, 154 ends and may be disposed between the bridge member 160 and the elongate shaft 110. In some embodiments the proximal ends 142, 152 of each leg 140, 150 may be coupled to the elongate shaft member 110 at its distal end 114.

In accordance with the present claimed invention, each leg 140, 150 forms substantially straight portions of the snare loop 130. The bridge member 160 extends between the two substantially straight legs 140, 150 in a rounded or generally arcuate shape.

The snare loop 130 is configured such that it has the same general shape whether it is fully or partially deployed from the outer sheath 120. For example, as shown by the arrow, and indicated by the dashed loop 130a, when the elongate shaft 110 is displaced in a proximal direction with respect to the outer sheath 120, portions of the snare loop 130 may be drawn into the outer sheath 120 through the distal opening 125. As the snare loop 130 is drawn into the outer sheath 120, the snare loop 130 may decrease in size, though it may retain its same general shape. For example, in the illustrated embodiment, both the snare loop 130 in the fully deployed position and the snare loop 130a illustrated in a partially deployed position, have substantially straight portions (e.g.legs 140, 150) extending from the distal opening 128 which are coupled to each other via a generally arcuate portion (e.g. bridge member 160).

In some embodiments, portions of the snare loop 130 which are rounded or arcuate when the snare loop 130 is fully deployed may become substantially straight as the snare loop 130 is drawn into the outer sheath 120. For example, the substantially straight portions of the partially deployed snare loop 130a may be portions of material which were arcuate when the snare loop was fully deployed, as illustrated by element 130. Similarly, as the partially deployed snare loop 130a is displaced distally with respect to the outer sheath 120, substantially straight portions may become arcuate. Thus, as referenced herein, the first leg 140, the second leg 150, and the bridge member 160 refer to the two straight portions and the arcuate portion of the snare loop 130, respectively; the same segment of material may be classified as either part of a leg portion or part of the bridge member depending on the position and disposition of the segment when the snare loop 130 is in a particular state of deployment. Additionally, any portion of the snare loop 130 constrained within the lumen 128 of the outer sheath 120 is referred to as a portion of either the first 140 or second 150 legs, notwithstanding it may not be positioned in a substantially straight line with the deployed portion of its respective leg.

Though in the embodiment of Figure 1A, the legs 140, 150 and other portions of the snare loop 130 may be constrained from radially expanding as the snare loop 130 is drawn into the outer sheath 120, the shape and characteristics of the snare loop 130 may not be dependent on the outer sheath 120. In other words, whether the snare loop 130 is partially or totally constrained by an outer sheath 120 or by some other device, mechanism, or force, the snare loop 130 may still exhibit the characteristics and features described above.

Figure 1B is another perspective view of the snare device of Figure 1A, oriented to show the position of the snare loop relative to the elongate shaft. In some embodiments, the snare loop 130 may be configured such that it does not lie in the same plane as the longitudinal axis of the outer sheath 120 when the snare loop 130 is deployed. In other embodiments the snare loop 130 may form a greater or smaller angle with respect to the axis of the outer sheath 120 as compared to the embodiment illustrated in Figure 1B. In certain embodiments, the snare loop 130 may be in the same plane as the longitudinal axis of the outer sheath 120, when the snare loop 130 is deployed.

Referring to both Figures 1A and 1B, in some embodiments the shape and disposition of the snare loop 130 may be controlled by forming the snare loop 130 from a shape memory alloy. In some embodiments, the snare loop 130 may be formed of nitinol. In other embodiments, the snare loop 130 may be formed in a particular shape or orientation, though it is not necessarily comprised of a shape memory or super elastic material.

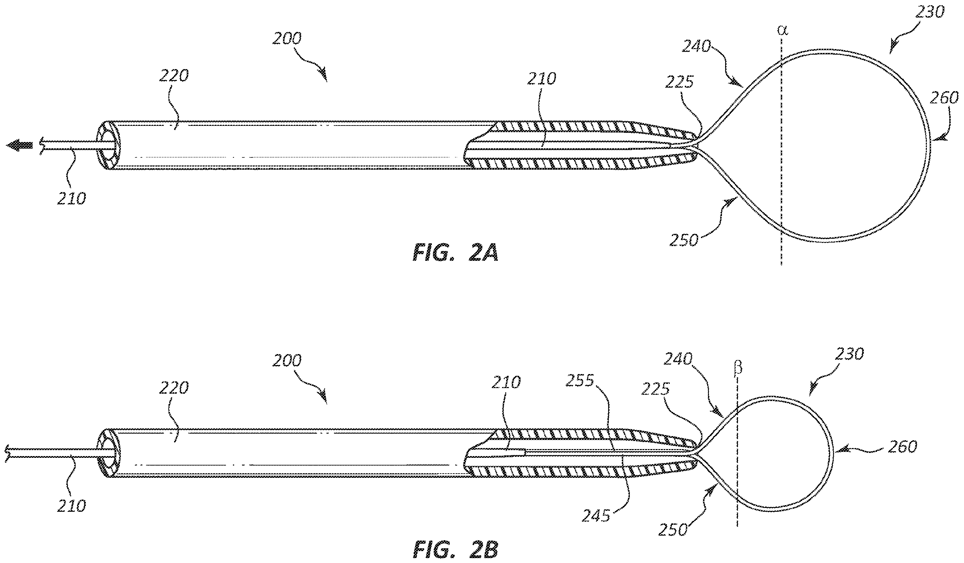

Figure 2A is a partial cut-away view of another embodiment of a variable loop snare device that can, in certain respects, resemble components of the snare device described in connection with Figures 1A and 1B above. It will be appreciated that all the illustrated embodiments may have analogous features. Accordingly, like features are designated with like reference numerals, with the leading digits incremented to "2." (For instance, the snare device is designated "100" in Figure 1A, and an analogous snare device is designated as "200" in Figure 2A.) Relevant disclosure set forth above regarding similarly identified features thus may not be repeated hereafter. Moreover, specific features of the snare device and related components shown in Figure 2A may not be shown or identified by a reference numeral in the drawings or specifically discussed in the written description that follows. However, such features may clearly be the same, or substantially the same, as features depicted in other embodiments and/or described with respect to such embodiments. Accordingly, the relevant descriptions of such features apply equally to the features of the snare device of Figure 2A. Any suitable combination of the features, and variations of the same, described with respect to the snare device and components illustrated in Figures 1A and 1B, can be employed with the snare device and components of Figure 2A, and vice versa. This pattern of disclosure applies equally to further embodiments depicted in subsequent figures and described hereafter.

Figure 2A is a partial cut-away view of another embodiment of a variable loop snare device 200 in a first configuration. In the configuration illustrated in Figure 2A, the snare loop 230 is substantially fully deployed from the outer sheath 220. By comparison, Figure 2B is a partial cut-away view of the snare device 200 of Figure 2A is a second configuration. In the configuration of Figure 2B, the snare loop 230 is partially retracted into the outer sheath 220 through the distal opening 225. The arrow in Figure 2A indicates how longitudinal displacement of the elongate shaft 210 in a proximal direction may retract the snare loop 230 through the distal opening 225, enabling a practitioner to transition the snare device 220 from the configuration of Figure 2A to the configuration of Figure 2B.

The snare loop 230 of Figures 2A and 2B comprises a first leg 240, a second leg 250, and a bridge member 260. As described above, the legs 240, 250 refer to substantially straight portions of the loop which extend from the distal opening 225 of the outer sheath 220. Further, portions of the snare loop 230 which are disposed within the outer sheath 220 (for example, proximal portions 245, 255 of Figure 2B) when the snare loop 230 is partially deployed are also referred to as portions of the first 240 and second 250 leg, respectively. Also as described above, in some instances, portions of the material comprising the snare loop 230 may be substantially straight (and thus classified as part of the legs) in some configurations of deployment, while the same segment of material may comprise a portion of the bridge segment (if it is generally arcuate in shape) in other configurations or positions of deployment. Further, any disclosure, related in connection with any embodiment, which describes the relative disposition and relationship of components of the snare device as the elongate shaft is displaced proximally, to scale down the snare loop size (or to retract the loop), is analogously applicable to the relationship and disposition of the components as the shaft is moved distally to scale up (or deploy), the snare loop. Similarly, disclosure relative to retracting the snare loop may analogously apply the reverse process of deploying the snare loop.

As illustrated in Figures 2A and 2B, the first 240 and second 250 legs form two adjacent legs of a substantially triangular shape disposed, in some embodiments, adjacent the distal opening 225 in the outer sheath 220. Lines a (alpha) and beta (beta) illustrate how the legs can be understood as forming two adjacent legs of these generally triangular shapes. In some embodiments, as the snare loop 230 is drawn into the outer sheath 220, generally arcuate portions of the bridge member 260 may transition and become substantially straight portions of the legs 240, 250, then be drawn into the outer sheath 220. As the legs 240, 250 are drawn into the outer sheath 220, proximal portions of each leg 245, 255 may be constrained to lie substantially adjacent within the outer sheath 220.

The generally triangular portion formed, in part, by the legs 240, 250, may form a transition portion of the snare loop 230, which may enable the snare loop 230 to maintain a generally open configuration - in some embodiments maintaining substantially the same general shape - as the snare loop 230 is drawn into, or deployed from, the outer sheath 220. For example, the substantially straight legs 240, 250 may form a transition portion near the distal opening 225 which limits the extent to which the bridge segment 260 of the snare loop 230 collapses, or deforms into an elongate shape when the snare loop 230 is retracted or deployed.

In the illustrated embodiment, both legs 240, 250 are coupled to a single elongate shaft 210. In another embodiment, two separate elongate shafts may be utilized, with a first elongate shaft coupled to the first leg 240 and a second elongate shaft coupled to the second leg 250. The use of two elongate shafts could allow a practitioner to displace both legs 240, 250 simultaneously or to displace one with respect to the other (i.e. pull one leg of the snare loop but not the other) to further control the size and shape of the snare loop.

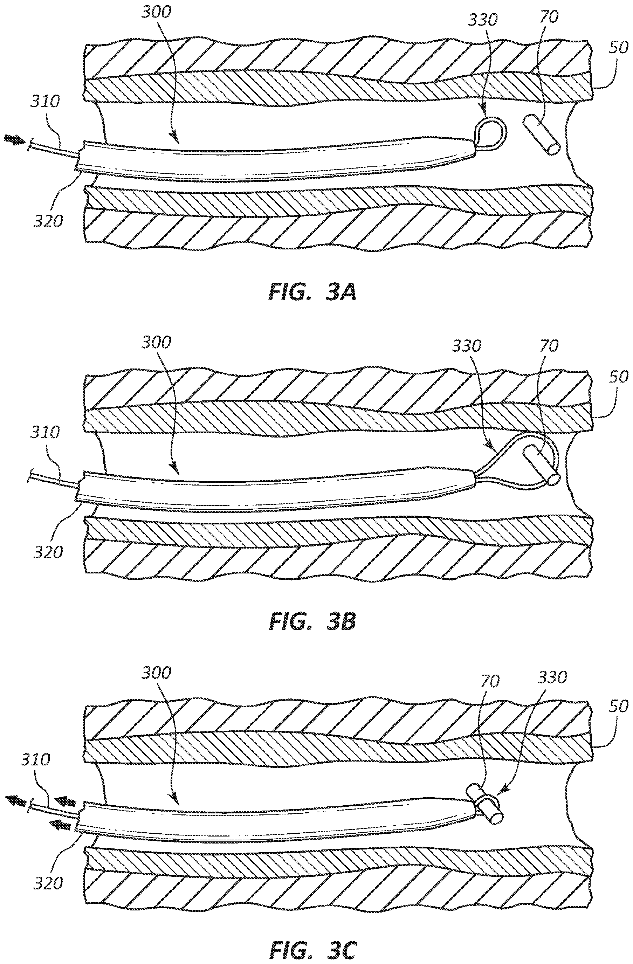

Figures 3A-3C are side views of a snare device 300, in three configurations, disposed within a cross-sectional view of a body lumen 50. These figures illustrate an exemplary procedure that may be performed utilizing a variable loop snare device, such as snare device 300. Figure 3A illustrates how a practitioner may deploy the snare loop 330 of the snare device 300 based on the parameters of the therapy. For example, the practitioner may attempt to capture a fragment 70 using the snare loop 330. Thus, the practitioner may displace the elongate shaft 310 is a distal direction with respect to the outer sheath 320 until the snare loop 330 is deployed such that it forms a loop having a desired size. The practitioner may then attempt to capture the fragment 70. At any point during the therapy the practitioner may adjust the size of the snare loop 330, larger or smaller, by displacing the elongate shaft 310 distally or proximally, depending on the parameters of the therapy.

Figure 3B illustrates how the fragment 70 may be enclosed by the snare loop 330 during therapy. Again, the practitioner may size and resize the snare loop 330 during the process in order to facilitate enclosure of the fragment by the snare loop 330. Figure 3C then illustrates how, in the exemplary procedure, the elongate shaft 310 may be displaced proximally with respect to the outer sheath 320 to close down the snare loop 330 and capture the fragment 70.
The examples and embodiments disclosed herein are to be construed as merely illustrative and exemplary, and not a limitation of the scope of the present disclosure in any way. It will be apparent to those having skill in the art, with the aid of the present disclosure, that changes may be made to the details of the above- described embodiments without departing from the underlying principles of the disclosure herein. It is intended that the scope of the invention be defined by the claims appended hereto.

## Claims

1. A snare device (100, 200, 300) comprising:
an elongate shaft (110, 210, 310), having a proximal end (112) and a distal end (114); and
a snare loop (130, 230, 330) coupled to the distal end (114) of the elongate shaft (110, 210, 310), the snare loop (130, 230, 330) comprising:
a first leg (140, 240), having a proximal end (142) and a distal end (144), the proximal end (142) coupled to the distal end (114) of the elongate shaft (110, 210, 310);
a second leg (150, 250) having a proximal end (152) and a distal end (154), the proximal end (152) coupled to the distal end (114) of the elongate shaft (110, 210, 310); and
a bridge segment (160, 260) coupled to and between the distal end (144) of the first leg (140, 240) and the distal end (154) of the second leg (150, 250);
wherein the snare loop (130, 230, 330) is configured to form an open loop when unconstrained, and the general shape of the open loop is the same whether the snare loop (130, 230, 330) is fully unconstrained or partially constrained
**characterized in that**
the first leg (140, 240) and the second leg (150, 250) form two adjacent legs of a generally triangular shape when the first (140, 240) and second (150, 250) legs are unconstrained, and
the bridge segment (160, 260) forms a generally arcuate shape.

2. The snare device (100, 200, 300) of claim 1, wherein the snare loop (130, 230, 330) has the same general shape whether the snare loop (130, 230, 330) is unconstrained or a proximal portion of the first leg (140, 240) is constrained to be disposed substantially adjacent a proximal portion of the second leg (150, 250).

3. The snare device (100, 200, 300) of claim 1, wherein unconstrained portions of the first leg (140, 240) and the second leg (150, 250) form two adjacent legs of a generally triangular shape when a proximal portion of the first leg (140, 240) is constrained to be disposed substantially adjacent a proximal portion of the second leg (150, 250).

4. The snare device (100, 200, 300) of claim 1, further comprising an elongate sheath member (120, 220, 320), the sheath member (120, 220, 320) comprising a proximal end, a distal end, a lumen disposed between the proximal and distal ends of the sheath member (120, 220, 320), and a distal opening in communication with the lumen,
wherein the elongate shaft is disposed within the lumen, and
wherein the snare loop (130, 230, 330) preferably forms a first open loop when fully deployed from the sheath member (120, 220, 320) and a second open loop when partially deployed from the sheath member (120, 220, 320), and wherein the first open loop and the second open loop have the same general shape.

5. The snare device (100, 200, 300) of claim 1, wherein the snare loop (130, 230, 330) is disposed in a different plane than the elongate shaft member when the snare loop (130, 230, 330) and elongate shaft member are unconstrained.

6. The snare device (100, 200, 300) of claim 1, wherein the snare loop (130, 230, 330) is comprised of a shape memory alloy.

## Patentansprüche

1. Ein Schlingenvorrichtung (100, 200, 300) aufweisend:
einen länglichen Schaft (110, 210, 310) mit einem proximalen Ende (112) und einem distalen Ende (114); und
eine Schlingenschlaufe (130, 230, 330), die mit dem distalen Ende (114) des länglichen Schafts (110, 210, 310) gekoppelt ist, wobei die Schlingenschlaufe (130, 230, 330) aufweist:
einen ersten Schenkel (140, 240) mit einem proximalen Ende (142) und einem distalen Ende (144), wobei das proximale Ende (142) mit dem distalen Ende (114) des länglichen Schafts (110, 210, 310) gekoppelt ist;
einen zweiten Schenkel (150, 250) mit einem proximalen Ende (152) und einem distalen Ende (154), wobei das proximale Ende (152) mit dem distalen Ende (114) des länglichen Schafts (110, 210, 310) gekoppelt ist; und
ein Brückensegment (160, 260), das mit und zwischen dem distalen Ende (144) des ersten Schenkels (140, 240) und dem distalen Ende (154) des zweiten Schenkels (150, 250) gekoppelt ist;
wobei die Schlingenschlaufe (130, 230, 330) eingerichtet ist eine offene Schlaufe zu bilden, wenn sie uneingeschränkt ist, und die allgemeine Form der offenen Schlaufe die gleiche ist unabhängig davon, ob die Schlingenschlaufe (130, 230, 330) vollständig uneingeschränkt oder teilweise eingeschränkt ist,
**dadurch gekennzeichnet, dass**
der erste Schenkel (140, 240) und der zweite Schenkel (150, 250) zwei benachbarte Schenkel von allgemein dreieckiger Form bilden, wenn der erste (140, 240) und der zweite (150, 250) Schenkel uneingeschränkt sind, und
das Brückensegment (160, 260) eine allgemein bogenförmige Form ausbildet.

2. Schlingenvorrichtung (100, 200, 300) nach Anspruch 1, wobei die Schlingenschlaufe (130, 230, 330) die gleiche allgemeine Form hat, unabhängig davon, ob die Schlingenschlaufe (130, 230, 330) uneingeschränkt ist oder ob ein proximaler Abschnitt des ersten Schenkels (140, 240) eingeschränkt ist, im Wesentlichen neben einem proximalen Abschnitt des zweiten Schenkels (150, 250) angeordnet zu sein.

3. Schlingenvorrichtung (100, 200, 300) nach Anspruch 1, wobei uneingeschränkte Abschnitte des ersten Schenkels (140, 240) und des zweiten Schenkels (150, 250) zwei benachbarte Schenkel mit einer im Allgemeinen dreieckigen Form bilden, wenn ein proximaler Abschnitt des ersten Schenkels (140, 240) eingeschränkt ist, im Wesentlichen neben einem proximalen Abschnitt des zweiten Schenkels (150, 250) angeordnet zu sein.

4. Schlingenvorrichtung (100, 200, 300) nach Anspruch 1, ferner aufweisend ein längliches Hülsenelement (120, 220, 320), wobei das Hülsenelement (120, 220, 320) ein proximales Ende, ein distales Ende, ein zwischen dem proximalen und dem distalen Ende des Hülsenelements (120, 220, 320) angeordnetes Lumen und eine distale Öffnung in Verbindung mit dem Lumen aufweist, wobei der längliche Schaft innerhalb des Lumens angeordnet ist, und
wobei die Schlingenschlaufe (130, 230, 330) vorzugsweise eine erste offene Schlaufe bildet, wenn sie vollständig aus dem Hüllenelement (120, 220, 320) entfaltet ist, und eine zweite offene Schlaufe, wenn sie teilweise aus dem Hüllenelement (120, 220, 320) entfaltet ist, und wobei die erste offene Schlaufe und die zweite offene Schlaufe die gleiche allgemeine Form haben.

5. Schlingenvorrichtung (100, 200, 300) nach Anspruch 1, wobei die Schlingenschlaufe (130, 230, 330) in einer anderen Ebene angeordnet ist als das längliche Schaftelement, wenn die Schlingenschlaufe (130, 230, 330) und das längliche Schaftelement uneingeschränkt sind.

6. Die Schlingenvorrichtung (100, 200, 300) nach Anspruch 1, wobei die Schlingenschlaufe (130, 230, 330) eine Formgedächtnislegierung aufweist.

## Revendications

1. Dispositif d'anse (100, 200, 300) comprenant :
une tige allongée (110, 210, 310), ayant une extrémité proximale (112) et une extrémité distale (114) ; et
une boucle d'anse (130, 230, 330) couplée à l'extrémité distale (114) de la tige allongée (110, 210, 310), la boucle d'anse (130, 230, 330) comprenant :
une première patte (140, 240), ayant une extrémité proximale (142) et une extrémité distale (144), l'extrémité proximale (142) étant couplée à l'extrémité distale (114) de la tige allongée (110, 210, 310) ;
une deuxième patte (150, 250) ayant une extrémité proximale (152) et une extrémité distale (154), l'extrémité proximale (152) étant couplée à l'extrémité distale (114) de la tige allongée (110, 210, 310) ; et
un segment de pont (160, 260) couplé à et entre l'extrémité distale (144) de la première patte (140, 240) et l'extrémité distale (154) de la deuxième patte (150, 250) ;
dans lequel la boucle d'anse (130, 230, 330) est configurée pour former une boucle ouverte lorsque non contrainte, et la forme générale de la boucle ouverte est la même que la boucle d'anse (130, 230, 330) soit totalement non contrainte ou partiellement contrainte
**caractérisé en ce que**
la première patte (140, 240) et la deuxième patte (150, 250) forment deux pattes adjacentes d'une forme généralement triangulaire lorsque les première (140, 240) et deuxième (150, 250) pattes sont non contraintes, et
le segment de pont (160, 260) est de forme généralement arquée.

2. Dispositif d'anse (100, 200, 300) selon la revendication 1, dans lequel la boucle d'anse (130, 230, 330) présente la même forme générale que la boucle d'anse (130, 230, 330) soit non contrainte ou qu'une partie proximale de la première patte (140, 240) soit contrainte à être disposée sensiblement adjacente à une partie proximale de la deuxième patte (150, 250).

3. Dispositif d'anse (100, 200, 300) selon la revendication 1, dans lequel les parties non contraintes de la première patte (140, 240) et de la deuxième patte (150, 250) forment deux pattes adjacentes d'une forme généralement triangulaire lorsqu'une partie proximale de la première patte (140, 240) est contrainte à être disposée sensiblement adjacente à une partie proximale de la deuxième patte (150, 250).

4. Dispositif d'anse (100, 200, 300) selon la revendication 1, comprenant en outre un élément de gaine allongée (120, 220, 320), l'élément de gaine (120, 220, 320) comprenant une extrémité proximale, une extrémité distale, une lumière disposée entre les extrémités proximale et distale de l'élément de gaine (120, 220, 320), et une ouverture distale en communication avec la lumière, dans lequel la tige allongée est disposée dans la lumière, et
dans lequel la boucle d'anse (130, 230, 330) forme préférentiellement une première boucle ouverte lorsque totalement déployée de l'élément de gaine (120, 220, 320) et une deuxième boucle ouverte lorsque partiellement déployée de l'élément de gaine (120, 220, 320), et dans lequel la première boucle ouverte et la deuxième boucle ouverte ont la même forme générale.

5. Dispositif d'anse (100, 200, 300) selon la revendication 1, dans lequel la boucle d'anse (130, 230, 330) est disposée dans un plan différent de l'élément de tige allongée lorsque la boucle d'anse (130, 230, 330) et l'élément de tige allongée sont non contraints.

6. Dispositif d'anse (100, 200, 300) selon la revendication 1, dans lequel la boucle d'anse (130, 230, 330) est constituée d'un alliage à mémoire de forme.
